# EUROPEAN PATENT APPLICATION

(11) **EP 4 578 484 A1**
(43) Date of publication of application: **02.07.2025**
(21) Application number: 23899679.7
(22) Date of filing: 07.11.2023
(51) Int. Cl.: A61M 60/17

(54) **DRIVING MECHANISM AND BLOOD PUMP**

(30) Priority: 09.12.2022 CN 202211580662
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YU, Shunzhou, Shenzhen, Guangdong 518000 (CN)
(74) Representative: Appleyard Lees IP LLP
(86) International application number: PCT/CN2023/130142
(87) International publication number: WO 2024/120098

(57) **Abstract**

A driving mechanism (10) and a blood pump (1). The driving mechanism (10) includes a housing assembly (11) and a rotating assembly (12). The housing assembly (11) is provided with a mounting groove (511) and a through hole (522). The mounting groove (511) has a bottom surface wall (512) and a side surface wall (513). The rotating assembly (12) has a far end and a near end. The far end of the rotating assembly (12) rotatably extends through the through hole (522). The near end of the rotating assembly (12) is provided with a rotating head (800). The rotating head (800) is rotatably arranged in the mounting groove (511) and slidably abuts against the bottom surface wall (512) and the side surface wall (513). The rotating head (800) is provided with a first spherical surface wall (810). The first spherical surface wall (810) is tangent to the side surface wall (513). The arrangement causes the driving mechanism and the blood pump structure to have a relatively long service life.

## Description

This application claims priority to Chinese Patent Application No. CN202211580662.1 filed with China National Intellectual Property Administration on December 9, 2022, the entire content of which is incorporated herein by reference.

### TECHNICAL FIELD

The present application relates to the field of medical instrument technologies, in particular to a driving mechanism and a blood pump.

### BACKGROUND

A blood pump is designed to be percutaneously inserted into a blood vessel of a patient such as a blood vessel of an artery or a vein in the thigh or the armpit, and can be advanced into the heart of the patient to function as a left ventricular assist device or a right ventricular assist device. Therefore, the blood pump can also be referred to as an intracardiac blood pump or an intravascular blood pump.

Generally, the blood pump has a driving mechanism and an impeller, and the impeller is connected to a rotating assembly of the driving mechanism. In order to realize stable rotation of the rotating assembly, a structure for positioning or limiting the rotating assembly is usually required to be provided, but a limiting end of the rotating assembly usually rotates relative to a housing to cause abrasion, so that abrasion resistance of the rotating assembly is difficult to maintain for long-time operation.

### SUMMARY

An object of the present application is to provide a driving mechanism and a blood pump which have a relatively long service life.

Embodiments of the present application provide a driving mechanism, the driving mechanism including:
a housing assembly including a mounting groove and a through hole, the mounting groove having a bottom surface wall and a side surface wall; and
a rotating assembly having a far end and a near end, the far end of the rotating assembly rotatably extending through the through hole, the near end of the rotating assembly including a rotating head, the rotating head being rotatably arranged in the mounting groove and slidably abutting against the bottom surface wall and the side surface wall, the rotating head having a first spherical surface wall, and the first spherical surface wall being tangent to the side surface wall.

Optionally, a cross section of the mounting groove along a direction perpendicular to a rotation axis of the rotating assembly is in a circular shape or a rectangular shape.

Optionally, a cross section of the side surface wall in a direction of a rotation axis of the rotating assembly is parallel to the rotation axis of the rotating assembly.

Optionally, the first spherical surface wall is tangent to the bottom surface wall, and/or the bottom surface wall is perpendicular to the side surface wall.

Optionally, the mounting groove further has a groove opening and an arc-shaped wall, the rotating head extends through the groove opening, the side surface wall connects the arc-shaped wall and the bottom surface wall, and the arc-shaped wall is adjacent to the groove opening.

Optionally, a distance from a tangent point between the first spherical surface wall and the side surface wall to a connecting line between the side surface wall and the bottom surface wall is 60% to 75% of a distance from the groove opening to the connecting line between the side surface wall and the bottom surface wall.

And/or, a distance from the tangent point between the first spherical surface wall and the side surface wall to a connecting line between the side surface wall and the arc-shaped wall is 0.01 mm to 0.015 mm.

Optionally, the rotating head includes a ball head portion and a rod portion connected to the ball head portion. The rod portion extends through the groove opening of the mounting groove. The ball head portion is rotatably received in the mounting groove. An avoidance section is formed at an end of the rod portion adjacent to the ball head portion, the avoidance section extends through the groove opening of the mounting groove, and a width of a cross section of the avoidance section in the direction perpendicular to the rotation axis of the rotating assembly is smaller than a diameter of the ball head portion.

Optionally, the width of the cross section of the avoidance section in the direction perpendicular to the rotation axis of the rotating assembly is 75% to 85% of a diameter of a sphere where the ball head portion is located.

Optionally, the rotating head includes a ball head portion, and the ball head portion extends through the groove opening of the mounting groove, a caliber of the groove opening of the mounting groove is smaller than or equal to the diameter of the sphere where the ball head portion is located and is greater than or equal to half of the diameter of the sphere where the ball head portion is located.

Optionally, the rotating head further has a second spherical surface wall, the second spherical surface wall is connected to the first spherical surface wall, the second spherical surface wall slidably abuts against the bottom surface wall of the mounting groove, and the second spherical surface wall is tangent to the bottom surface wall.

Optionally, a convex ring is further arranged on the rotating head, a central axis of the convex ring coincides with the rotation axis of the rotating assembly, and the second spherical surface wall is arranged on the convex ring.

Optionally, an inner diameter of the convex ring is not smaller than half of a diameter of a sphere where the first spherical surface wall is located.

Optionally, the housing assembly includes a pump case and a support member mounted on the pump case. The mounting groove is formed in the support member. The driving mechanism further includes a mounting base. The mounting base is fixedly connected to the pump case. A mounting cavity and a liquid passing hole communicated with the mounting cavity are formed in the mounting base. The support member is mounted in the mounting cavity. A flow channel is formed in at least one of the mounting base and the support member. The flow channel is communicated with the liquid passing hole.

Optionally, the flow channel is formed in the support member, the flow channel in the support member has a first opening and a second opening, the first opening is communicated with the mounting groove, the second opening is communicated with the liquid passing hole, and the rotating head avoids the first opening.

Optionally, the support member includes a collar and a bottom plate. The bottom plate is connected to an end of the collar. The bottom plate and the collar together define the mounting groove. At least a part of an inner wall of the collar forms the side surface wall, and the bottom surface wall is arranged on the bottom plate.

Optionally, the bottom plate is provided with the flow channel communicated with the mounting groove, the flow channel in the bottom plate extends from a side surface of the bottom plate towards a center of the bottom plate, and the rotating head avoids the flow channel in the bottom plate.

Optionally, the rotating assembly further includes a rotating shaft and a rotor. The rotating shaft has a near end and a far end. The far end of the rotating shaft rotatably extends through the through hole. The rotor includes a first rotor unit. The first rotor unit is fixedly connected to the rotating shaft. At least one of the first rotor unit and the rotating shaft is connected to the rotating head.

Optionally, the rotating head includes a stop surface, the stop surface is located on a side of the rotating head away from the bottom surface wall of the mounting groove, a distance exists between the stop surface and the groove opening of the mounting groove, and the first rotor unit abuts against the stop surface, so that the first rotor unit is spaced a distance from the groove opening of the mounting groove.

Optionally, the housing assembly includes a pump case and a shaft sleeve mounted in the pump case. The through hole is formed in the shaft sleeve. The driving mechanism further includes a stop member. The stop member is fixedly connected to the rotating assembly, the stop member is located between the shaft sleeve and the rotating head, and the stop member can abut against the shaft sleeve to restrict the rotating assembly from moving away from the mounting groove.

Embodiments of the present application further provide a blood pump, including an impeller and the driving mechanism in any of the above, the impeller being connected to the rotating assembly, and the impeller rotating with the rotating assembly.

According to the driving mechanism of the present solution, the rotating head abuts against the side surface wall and the bottom surface wall of the mounting groove to limit the near end of the rotating assembly. The first spherical surface wall of the rotating head is tangent to the side surface wall. When the rotating head rotates, the contact between the rotating head and the side surface wall of the mounting groove can be regarded as line-to-line contact. Compared with the fitting mode that the whole first spherical surface wall of the rotating head is in contact with a groove wall of a ball head groove, a contact area between the rotating head and the groove wall is reduced. That is, an abrasion area between the rotating head and the side surface wall is small, abrasion resistance of the rotating head is better, and the service life is longer. Meanwhile, since the contact area is small, friction resistance is also small when the rotating head is just started; the torque at starting is smaller, and the starting is faster.

### BRIEF DESCRIPTION OF THE DRAWINGS

In order to illustrate the technical solutions in the embodiments of the present application more clearly, the drawings required for describing the embodiments or the prior art will be described briefly. Apparently, the following described drawings are merely for some embodiments of the present application, and other drawings can be derived from these drawings by those of ordinary skill in the art without any creative effort.
FIG. 1 is a schematic structural view of a blood pump according to an embodiment of the present application.
FIG. 2 is a sectional view of the blood pump shown in FIG. 1 with a part of a catheter omitted.
FIG. 3 is a first partial enlarged view of portion I shown in FIG. 2.
FIG. 4 is a second partial enlarged view of portion I shown in FIG. 2.
FIG. 5 is a third partial enlarged view of portion I shown in FIG. 2.
FIG. 6 is a partial enlarged structural view of a far end part of the blood pump shown in FIG. 2.
FIG. 7 is a schematic structural view of a support member of the blood pump shown in FIG. 2.
FIG. 8 is a schematic structural view of a mounting base of the blood pump shown in FIG. 2.
FIG. 9 is a partial enlarged structural view of a near end part of the blood pump shown in FIG. 2.
FIG. 10 is a schematic structural view of a shaft sleeve in FIG. 2.
FIG. 11 is a partial enlarged structural view of a far end part of a blood pump according to a second embodiment of the present application.
FIG. 12 is a partial enlarged view of portion II of FIG. 11.
FIG. 13 is a partial enlarged structural view of a far end part of a blood pump according to a third embodiment of the present application.
FIG. 14 is a schematic structural view of a rotating head in FIG. 13.
FIG. 15 is a schematic structural view of a support member in FIG. 13.
FIG. 16 is a schematic structural view of the support member in FIG. 13 formed by a collar and a bottom plate.

### DETAILED DESCRIPTION

Embodiments of the present application are described in detail below, examples of which are illustrated in the accompanying drawings, wherein the same or similar reference numerals refer to the same or similar elements or elements having the same or similar functions throughout. The embodiments described below with reference to the accompanying drawings are exemplary and intended to explain the present application and should not be construed as limiting the present application.

Reference throughout this specification to "one embodiment" or "an embodiment" means that a particular feature, structure, or characteristic described in connection with the embodiment is included in at least one embodiment of the present application. Therefore, the appearances of the phrases "in one embodiment" or "in some embodiments" in various places throughout this specification are not necessarily all referring to the same embodiment. In addition, in one or more embodiments, particular features, structures, or characteristics can be combined in any suitable manner.

In descriptions of the present application, it should be understood that, directions or positional relationships indicated by terms "length", "width", "upper", "lower", "front", "rear", "left", "right", "vertical", "horizontal", "top", "bottom", "inner", "outer", etc. are based on orientations or positional relationships shown in the accompanying drawings, and they are used only for describing the present application and for description simplicity, but do not indicate or imply that an indicated device or element must have a specific orientation or be constructed and operated in a specific orientation. Therefore, it cannot be understood as a limitation on the present application.

In addition, the terms such as "first" and "second" are used herein for purposes of description and are not intended to indicate or imply relative importance or significance or to imply the number of indicated technical features. Thus, the feature defined with "first" or "second" can include one or more of said feature explicitly or implicitly.

In the present application, unless specified or limited otherwise, the terms "mounted", "connected", "coupled", and "fixed" and the like are used broadly, and can be, for example, fixed connections, detachable connections, or integral connections; can also be mechanical or electrical connections; can also be direct connections or indirect connections via intervening structures; can also be communication or an interaction relationship of two elements. The above terms can be understood by those skilled in the art according to specific situations.

In the field of interventional medical treatment, it is common to define the end of an instrument adjacent to an operator as the near end and the end of an instrument away from the operator as the far end.

A driving mechanism 10 and a blood pump 1 according to embodiments of the present application are described.

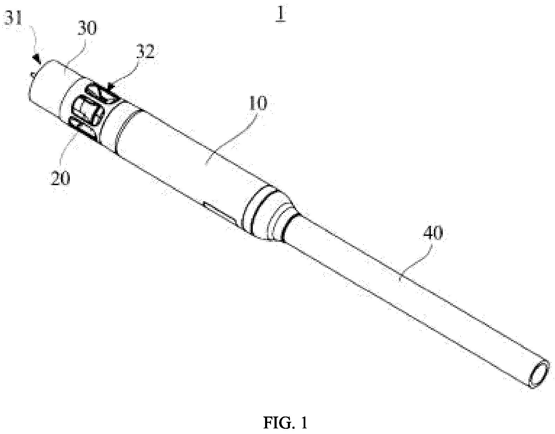

Referring to FIG. 1, the blood pump 1 includes a driving mechanism 10 and an impeller 20, the driving mechanism 10 is in transmission connection to the impeller 20, and the driving mechanism 10 can drive the impeller 20 to rotate.

Specifically, the blood pump 1 further includes a cannula 30, the cannula 30 being connected to the driving mechanism 10. The impeller 20 is rotatably received in the cannula 30. The cannula 30 has a blood inlet 31 and a blood outlet 32. When the impeller 20 rotates, blood flows into the cannula 30 from the blood inlet 31 and then flows out from the blood outlet 32. When the blood pump 1 is used in the left ventricle, the cannula 30 extends through a heart valve such as an aortic valve. The blood inlet 31 is located inside the ventricle of the heart. The blood outlet 32 and the driving mechanism 10 are located in a blood vessel such as the aorta outside the heart.

Specifically, the blood pump 1 further includes a catheter 40, the catheter 40 being connected to the driving mechanism 10. The catheter 40 is configured to receive various supply lines. More specifically, the supply lines include an electric wire for being electrically connected to the driving mechanism 10, a signal wire for being electrically connected to a sensor in the blood pump 1, and a flushing line for injecting flushing liquid into the blood pump 1. For example, the flushing liquid can be physiological saline, physiological saline containing heparin, glucose, or the like.

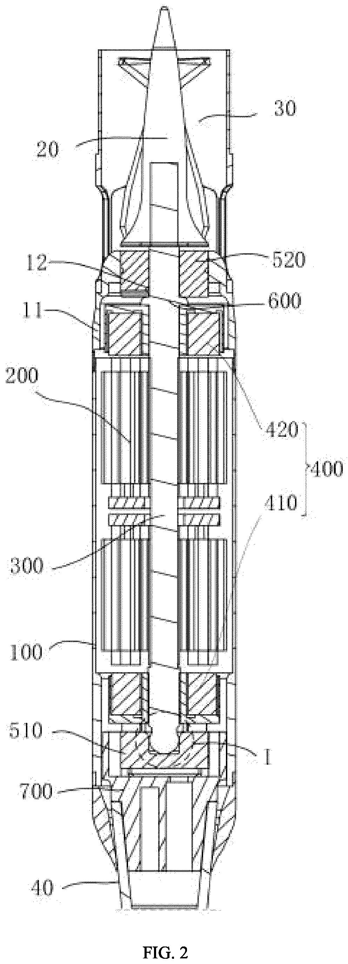

Referring to FIG. 2 to FIG. 6, the driving mechanism 10 includes a housing assembly 11 and a rotating assembly 12. The housing assembly 11 includes a mounting groove 511 and a through hole 522. The mounting groove 511 has a bottom surface wall 512 and a side surface wall 513. The rotating assembly 12 has a far end and a near end. The far end of the rotating assembly 12 rotatably extends through the through hole 522. The near end of the rotating assembly 12 includes a rotating head 800. The rotating head 800 is rotatably arranged in the mounting groove 511 and slidably abuts against the bottom surface wall 512 and the side surface wall 513. The rotating head 800 has a first spherical surface wall 810. The first spherical surface wall 810 is tangent to the side surface wall 513. The cannula 30 and the catheter 40 are both fixedly connected to the housing assembly 11. A part of the rotating assembly 12 is located inside the cannula 30, and the other part of the rotating assembly 12 is located inside the housing assembly 11. The impeller 20 is fixedly connected to the far end of the rotating assembly 12.

In the driving mechanism, the rotating head 800 abuts against the side surface wall 513 and the bottom surface wall 512 of the mounting groove 511 to limit the near end of the rotating assembly 12. The first spherical surface wall 810 of the rotating head 800 is tangent to the side surface wall 513. When the rotating head 800 rotates, the contact between the rotating head 800 and the side surface wall 513 of the mounting groove 511 can be regarded as line-to-line contact. Compared with the fitting mode that the whole first spherical surface wall 810 of the rotating head 800 is in contact with a groove wall of a ball head groove, a contact area between the rotating head 800 and the groove wall of the mounting groove 511 is reduced. That is, an abrasion area between the rotating head 800 and the side surface wall 513 is small, abrasion resistance of the rotating head 800 is better, and a service life is longer. Meanwhile, since the contact area is small, friction resistance is also small when the rotating head 800 is just started; the torque at starting is smaller, and the starting is faster.

In the illustrated embodiment, the side surface wall 513 is parallel to a rotation axis of the rotating assembly 12 when the rotating assembly 12 is rotated stably. Since the rotating assembly 12 may undergo a small amplitude of axial movement or radial deflection during rotation, with this arrangement, when the rotating assembly 12 axially moves in the mounting groove 511, no matter where the rotating assembly moves, the rotating head 800 can keep abutting against the side surface wall 513, with stable radial limitation; when the rotating assembly 12 is radially deflected, the rotating head 800 can roll in the mounting groove 511, and the first spherical surface wall 810 is kept to be tangent to the side surface wall 513 at all times.

The stable rotation of the rotating assembly 12 means that when the rotating assembly 12 rotates, the rotation axis of the rotating assembly 12 substantially coincides with a central axis of the housing assembly 11.

Certainly, in other embodiments, the side surface wall 513 can be inclined at a certain angle relative to the rotation axis of the rotating assembly 12. Specifically, the included angle between the side surface wall 513 and the rotation axis of the rotating assembly 12 is an acute angle. That is, a distance between the side surface wall 513 and the rotation axis of the rotating assembly 12 gradually increases in a direction away from the bottom surface wall 512, so that the rotating head 800 more smoothly enters the mounting groove 511 during assembly.

The mounting groove 511 has a certain depth, and a cross section of the mounting groove 511 along a direction perpendicular to the rotation axis of the rotating assembly 12 is in a circular shape. In the illustrated embodiment, the side surface wall 513 is a cylindrical surface. When the rotating head 800 abuts against the side surface wall 513, the first spherical surface wall 810 is tangent to the side surface wall 513. That is, when the rotating head 800 rotates, the contact between the first spherical surface wall 810 and the side surface wall 513 can be considered a line-to-line contact, so that the contact area can be reduced. Meanwhile, when the rotating head 12 is radially deflected, the side surface wall 513 being the cylindrical surface can be tangent to the rotating head 800 in a line-to-line contact mode no matter which position the rotating head 800 rolls to, thereby maintaining a radial limiting effect on the rotating head 800.

In some embodiments, alternatively, the cross section of the mounting groove 511 can be rectangular. That is, the side surface wall 513 is defined by four planes. When the rotating head 800 abuts against the side surface wall 513, the first spherical surface wall 810 is tangent to each of the four planes. When the rotating head 800 rotates, the contact between the first spherical surface wall 810 and the side surface wall 513 can be regarded as a point-to-point contact, so that such an arrangement can also reduce the contact area, thereby reducing abrasion of the rotating head 800. In other embodiments, alternatively, the cross section of the mounting groove 511 can be triangular, pentagonal, hexagonal, or the like, and the point-to-point contact can also be formed to reduce the contact area, thereby reducing the abrasion.

As shown in FIG. 2, FIG. 3, FIG. 9, and FIG. 10 together, the housing assembly 11 includes a pump case 100, a support member 510, and a shaft sleeve 520.

Specifically, the pump case 100 is substantially in a cylindrical structure with both ends having openings. A far end of the pump case 100 is fixedly connected to the cannula 30, and a near end of the pump case 100 is fixedly connected to the catheter 40.

The support member 510 and the shaft sleeve 520 are both mounted in the pump case 100. The support member 510 is arranged at the near end of the pump case 100. The shaft sleeve 520 is arranged at the far end of the pump case 100. The support member 510 and the shaft sleeve 520 are fixedly connected to the pump case 100. Specifically, the mounting groove 511 is formed in the support member 510. The through hole 522 is formed in the shaft sleeve 520.

A central axis of the through hole 522 coincides with a central axis of the mounting groove 511. The far end of the rotating assembly 12 rotatably extends through the through hole 522. A gap for fluid flow is provided between a hole wall of the through hole 522 of the shaft sleeve 520 and the rotating assembly 12. The flushing liquid entering the pump case 100 can flow out of the pump case 100 through the gap between the rotating assembly 12 and the hole wall of the through hole 522.

The rotating assembly 12 further includes a rotating shaft 300 and a rotor 400. A rotation axis of the rotating shaft 300 coincides with the rotation axis of the rotating assembly 12. That is, when the rotating assembly 12 stably rotates, the rotation axis of the rotating shaft 300 substantially coincides with the central axis of the housing assembly 11. The rotating shaft 300 is rotatably mounted in the pump case 100. The rotating shaft 300 has a connecting end 310 configured to be connected to the impeller 20, so that the impeller 20 can rotate with the rotating shaft 300. In the illustrated embodiment, the rotating shaft 300 extends through the through hole 522 of the shaft sleeve 520. The rotating shaft 300 extends substantially along an axial direction of the pump case 100. The connecting end 310 of the rotating shaft 300 extends outside the pump case 100, alternatively, extends into the cannula 30 to be connected to the impeller 20. The rotation axis of the rotating assembly 12 is the rotation axis of the rotating shaft 300. The rotor 400 is arranged inside the pump case 100, and the rotor 400 is fixedly connected to the rotating shaft 300.

The driving mechanism further includes a stator 200, and the stator 200 is arranged inside the pump case 100. The stator 200 can drive the rotating assembly 12 to rotate. In the illustrated embodiment, the stator 200 is located between the support member 510 and the shaft sleeve 520.

The stator 200 can drive the rotor 400 to rotate, and the rotor 400 can drive the rotating shaft 300 to rotate. Specifically, the rotor 400 has magnetism, and the stator 200 can generate a rotating magnetic field for driving the rotor 400 to rotate. The rotating shaft 300 rotatably extends through the stator 200. The rotor 400 is located between the support member 510 and the shaft sleeve 520.

Referring to FIG. 2 again, in the illustrated embodiment, the rotor 400 includes a first rotor unit 410 and a second rotor unit 420, and the first rotor unit 410 and the second rotor unit 420 are both fixedly connected to the rotating shaft 300. The first rotor unit 410 and the second rotor unit 420 are arranged along the axis of the rotating shaft 300. The stator 200 is located between the first rotor unit 410 and the second rotor unit 420. Both the first rotor unit 410 and the second rotor unit 420 have magnetism. The stator 200 can generate a rotating magnetic field for driving the first rotor unit 410 and the second rotor unit 420 to rotate. At least one of the rotating shaft 300 and the first rotor unit 410 is connected to the rotating head 800. In the illustrated embodiment, the rotating head 800 is fixedly connected to an end of the rotating shaft 300 away from the connecting end 310.

The rotating head 800 and the rotating shaft 300 can be split, and the rotating head 800 can be fixedly connected to the rotating shaft 300 and the first rotor unit 410 by welding, bonding, or the like. In some embodiments, alternatively, the rotating head 800 and the rotating shaft 300 can be formed as an integral structure, which is convenient for processing and forming. Alternatively, in other embodiments, the rotating head 800 cannot be directly connected to the rotating shaft 300. For example, the rotating head 800 is connected to the first rotor unit 410.

The driving mechanism further includes a stop member 600. The stop member 600 is fixedly connected to the rotating assembly 12. The stop member 600 is located between the shaft sleeve 520 and the rotating head 800. The stop member 600 can abut against the shaft sleeve 520 to restrict the rotating assembly 12 from moving away from the mounting groove 511.

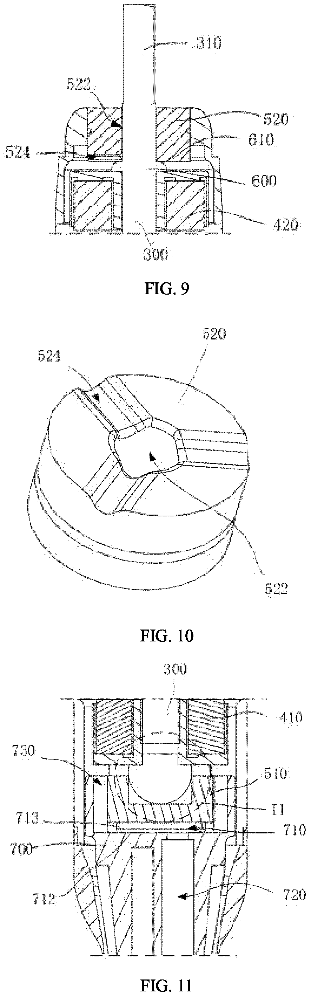

As shown in FIG. 9 and FIG. 10, specifically, the stop member 600 is fixedly connected to at least one of the rotating shaft 300 and the rotor 400 (specifically, the second rotor unit 420). In other words, the stop member 600 can be directly fixed only to the rotor 400. Alternatively, the stop member 600 can be directly fixed only to the rotating shaft 300. Alternatively, the stop member 600 can be directly fixed to both the rotor 400 and the rotating shaft 300. Since the rotor 400 is fixedly connected to the rotating shaft 300, the stop member 600, the rotating shaft 300, and the rotor 400 rotate and move synchronously. The stop member 600 is located between the rotor 400 and the shaft sleeve 520, and the stop member 600 can abut against the shaft sleeve 520 to restrict the rotating shaft 300 from moving away from the mounting groove 511 along the axis of the rotating shaft 300.

In the illustrated embodiment, the stop member 600 and the rotating shaft 300 are integrally formed. Since the blood pump 1 has a small overall size, the stop member 600 has a smaller size, resulting in difficulty in machining precision and large assembly difficulty. When the stop member 600 and the rotating shaft 300 are integrally formed, mounting is convenient, and a fixed connecting operation is omitted.

Specifically, the stop member 600 is substantially annular, and a central axis of the stop member 600 coincides with the axis of the rotating shaft 300. The stop member 600 has a stop surface 610, and the stop surface 610 is an arc-shaped surface, so that a contact area between the stop surface and the shaft sleeve 520 can be reduced, and abrasion between the stop surface and the shaft sleeve is reduced. The stop surface 610 abuts against the shaft sleeve 520 to restrict movement of the rotating shaft 300 towards the impeller 20 along the axis of the rotating shaft 300.

A surface of the shaft sleeve 520 facing the stop member 600 is partially recessed to form a flow guide groove 524, and the flow guide groove 524 is communicated with the through hole 522 of the shaft sleeve 520. When the stop member 600 abuts against the shaft sleeve 520, a part of the flow guide groove 524 is not covered by the stop member 600, so that when the stop member 600 abuts against the shaft sleeve 520, even if the stop member 600 blocks the gap between the through hole 522 of the shaft sleeve 520 and the rotating shaft 300 to cause a problem of flow obstruction of the flushing liquid, the flow guide groove 524 which is not covered by the stop member 600 can realize fluid communication when the stop member 600 abuts against the shaft sleeve 520, thereby ensuring the smoothness of flow of the flushing liquid. In addition, the flow guide groove 524 is formed by partially recessing the surface of the shaft sleeve 520 facing the stop member 600, so that the flushing liquid can better flow into a space between the stop member 600 and the shaft sleeve 520, thereby playing a role of lubricating the contact surface between the stop member 600 and the shaft sleeve 520, thereby reducing the friction between the stop member 600 and the shaft sleeve 520, and reducing the abrasion caused by the friction between the stop member 600 and the shaft sleeve 520.

In the illustrated embodiment, three flow guide grooves 524 are provided. The three flow guide grooves 524 are communicated with the through hole 522 and are uniformly distributed relative to the through hole 522. Due to existence of the three flow guide grooves 524, more flushing fluid can be introduced, so that the contact surface between the stop surface 610 and the shaft sleeve 520 is better lubricated and friction is reduced. Meanwhile, the contact area between the shaft sleeve 520 and the stop surface 610 is also reduced, and the abrasion is reduced.

Certainly, in other embodiments, the number of the flow guide groove 524 can be one, two, or more than three, which is not limited herein.

The following embodiments specifically illustrate the structure of the rotating head 800.

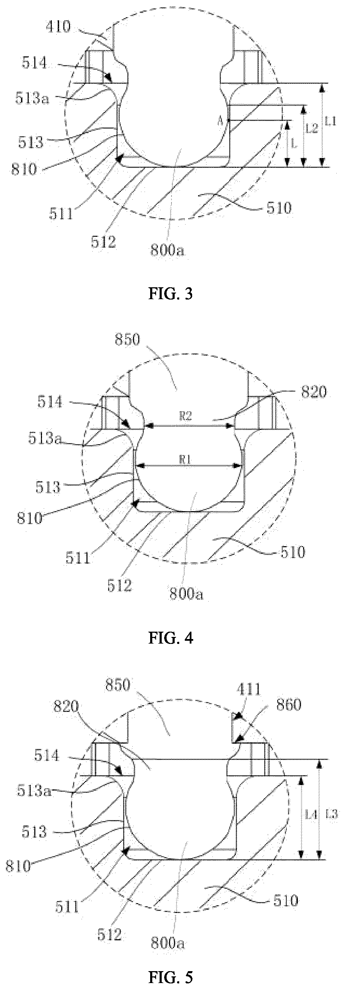

First embodiment: as shown in FIG. 3, the rotating head 800 includes a ball head portion 800a, the ball head portion 800a is rotatably arranged in the mounting groove 511, and the ball head portion 800a slidably abuts against the bottom surface wall 512 and the side surface wall 513. Specifically, in the illustrated embodiment, the first spherical surface wall 810 of the ball head portion 800a is tangent to both the side surface wall 513 and the bottom surface wall 512. The side surface wall 513 of the mounting groove 511 is a cylindrical surface, the bottom surface wall 512 is a plane, and the bottom surface wall 512 is perpendicular to the side surface wall 513. The first spherical surface wall 810 is at least half of a spherical surface of a sphere where the first spherical surface wall 810 is located, such that a volume of the ball head portion 800a is at least half of the sphere where the first spherical surface wall 810 is located. A tangent point between the first spherical surface wall 810 and the side surface wall 513 is located at the cross section of the maximum diameter of the ball head portion 800a, and the diameter of the maximum cross section is equal to the diameter of the sphere where the ball head portion 800a is located. When the rotating head 800 rotates, the first spherical surface wall 810 and the side surface wall 513 are in a line-to-line contact, and the contact area is small, so that the abrasion can be reduced. The tangent point between the first spherical surface wall 810 and the bottom surface wall 512 can be regarded as a point-to-point contact, which can reduce friction and abrasion between the first spherical surface wall and the bottom surface wall 512. In addition, abutting of the ball head portion 800a against the bottom surface wall 512 can also realize an axial limitation on the near end of the rotating assembly 12. The ball head portion 800a is received in the mounting groove 511, which contributes to a stable radial limitation on and smoother rolling of the mounting groove 511.

In this embodiment, the volume of the ball head portion 800a is greater than half of the sphere where the first spherical surface wall 810 is located. In this way, it is possible to prevent the side surface wall 513 of the mounting groove 511 from being scratched and worn due to the contact of the side surface wall 513 of the mounting groove 511 with the included angle formed at the connection between the surface of the ball head portion 800a away from the bottom surface wall 512 of the mounting groove 511 and the first spherical surface wall 810.

More specifically, the mounting groove 511 further has a groove opening 514 and an arc-shaped wall 513a, the rotating head 800 extends through the groove opening 514, the arc-shaped wall 513a is arranged adjacent to the edge of the groove opening 514 and extends away from the central axis of the groove opening 514, and the side surface wall 513 is connected to the arc-shaped wall 513a and the bottom surface wall 512. In this embodiment, the arc-shaped wall 513a has a rounded structure arranged at the edge of the groove opening 514. During rotation of the rotating assembly 12, the far end of the rotating assembly 12 can vibrate to cause the rotating head 800 at the near end of the rotating assembly 12 to be radially deflected, so that the rotating head 800 can swing away from the rotation axis of the stably-rotating rotating assembly 12, resulting in the risk that the rotating head 800 is jammed by the mounting groove 511. When the arc-shaped wall 513a (i.e., a rounded structure) is arranged at the groove opening 514, a caliber of the groove opening 514 can be enlarged relatively and the wall surface can be smooth, so that on the one hand, a space for the radial deflection of the rotating head 800 can be provided to reduce the risk of jamming of the rotating head 800, and on the other hand, the risk of scratching and abrasion of the rotating head 800 by the edge of the opening of the mounting groove that is not rounded during the radial deflection can be lowered.

As shown in FIG. 3, when the rotating assembly 12 rotates stably, in the direction of the rotation axis of the rotating assembly 12, a distance from the tangent point A between the first spherical surface wall 810 and the side surface wall 513 to the connecting line between the side surface wall 513 and the bottom surface wall 512 is 60% to 75% of a distance from the groove opening 514 to the connecting line between the side surface wall 513 and the bottom surface wall 512. Specifically, the tangent point A between the first spherical surface wall 810 and the side surface wall 513 is an abutting point between the ball head portion 800a and the side surface wall 513. By arranging the abutting point as described above, the risk that the rotating head 800 axially moves to get out of the mounting groove 511 during rotation can be reduced.

In this embodiment, the bottom surface wall 512 is a plane, the bottom surface wall 512 is perpendicular to the side surface wall 513, and the first spherical surface wall 810 is tangent to the bottom surface wall 512 and the side surface wall 513, so that the distance from the tangent point A between the first spherical surface wall 810 and the side surface wall 513 to the connecting line between the side surface wall 513 and the bottom surface wall 512 is a radius of the sphere where the ball head portion 800a is located, and defined as L; the distance from the groove opening 514 to the connecting line between the side surface wall 513 and the bottom surface wall 512 is a depth of the mounting groove 511 and defined as L1. Therefore, 60%≤L/L1≤75%.

Specifically, the distance from the tangent point A between the first spherical surface wall 810 and the side surface wall 513 to the connecting line between the side surface wall 513 and the arc-shaped wall 513a is 0.01 mm to 0.015 mm. A distance from the connecting line between the side surface wall 513 and the arc-shaped wall 513a to the connecting line between the side surface wall 513 and the bottom surface wall 512 is defined as L2. That is, a difference between L2 and L is between 0.01 mm and 0.015 mm. With such an arrangement, on one hand, the mounting groove 511 can have a proper depth, and meanwhile, the risk that the rotating head 800 moves to get out of the mounting groove 511 can be reduced; on the other hand, the risk of the rotary head 800 being out of abutting against the side surface wall 513 of the mounting groove 511 due to the movement of the rotating head 800 can be reduced.

As shown in FIG. 4, the ball head portion 800a can be rotatably received in the mounting groove 511, and the radius of the sphere where the ball head portion 800a is located is not greater than the depth of the mounting groove 511, so that the ball head portion 800a is completely arranged in the mounting groove 511, which can increase stability of the rotation of the ball head portion 800a.

The rotating head 800 further includes a rod portion 850 connected to the ball head portion 800a. The rod portion 850 extends through the groove opening 514 of the mounting groove 511. Specifically, an end of the rod portion 850 away from the ball head portion 800a is connected to the rotating shaft 300 or the first rotor unit 410. Specifically, in the illustrated embodiment, an end of the rod portion 850 away from the ball head portion 800a is fixedly connected to the rotating shaft 300 (specifically, an end of the rotating shaft 300 away from the connecting end 310). In this embodiment, the rod portion 850 is a cylindrical segment similar to the rotating shaft 300, and the rotating shaft 300, the rod portion 850 and the ball head portion 800a are integrally formed.

In order to further reduce the risk that the rotating head 800 is jammed or collides with the edge of the groove opening 514 of the mounting groove 511 during the radial deflection, an avoidance section 820 is formed at an end of the rod portion 850 adjacent to the ball head portion 800a, the avoidance section 820 extends through the groove opening 514 of the mounting groove 511, and a width of a cross section of the avoidance section 820 in the direction perpendicular to the rotation axis of the rotating shaft 300 is smaller than the diameter of the sphere where the ball head portion 800a is located. The rotation axis of the rotating shaft 300 is the rotation axis of the rotating assembly 12.

In a state when the rotating assembly 12 is not started, a cross section of the side surface wall 513 in the axial direction of the rotating shaft 300 is parallel to the rotation axis of the rotating shaft 300, and the diameter of the sphere where the ball head portion 800a is located is also the width of the mounting groove 511. In this embodiment, the diameter of the sphere where the ball head portion 800a is located is defined as R1, and R1 is also the width of the mounting groove 511. A diameter of the avoidance section 820 is defined as R2. Therefore, R1<R2.

When the rotating assembly 12 is deflected in a rotating state, the ball head portion 800a can roll in the mounting groove 511. Since the diameter of the avoidance section 820 is smaller than the width of the mounting groove 511, in the deflecting process, a distance between the rod portion 850 and the groove opening 514 is increased due to the existence of the avoidance section 820, the rod portion 850 can have a larger deflecting angle, an upper limit of the deflecting angle is improved, thereby adapting a larger radial deflecting distance.

Still further, under the condition of satisfying the adaptive deflection, the diameter of the avoidance section 820 cannot be excessively small, otherwise, the avoidance section is excessively thin, has insufficient strength and is prone to breakage. The diameter of the avoidance section 820 is set to be 75% to 85% of the diameter of the sphere where the ball head portion 800a is located, that is, 75%≤R1/R2≤85%, so that the avoidance section 820 has the effects of adapting to the deflection and reducing the risk of collision with the edge of the groove opening 514, and the strength of the avoidance section 820 can meet requirements.

As shown in FIG. 5, in the direction of the rotation axis of the rotating shaft 300, the avoidance section 820 has a certain axial length, and a distance from an end of the avoidance section 820 away from the ball head portion 800a to an end of the ball head portion 800a away from the avoidance section 820 is not less than the depth of the mounting groove 511. In this embodiment, the depth of the mounting groove 511 is defined as L4, the rotating shaft 300 is connected to the first rotor unit 410, the rod portion 850 is also connected to the first rotor unit 410, the avoidance section 820 is arranged between the first rotor unit 410 and the support member 510, and the avoidance section 820 extends from the interior of the mounting groove 511 towards the first rotor unit 410 and has a certain length. The distance from the end of the avoidance section 820 away from the ball head portion 800a to the end of the ball head portion 800a away from the avoidance section 820 is defined as L3, and L3>L4, so that when the ball head portion 800a abuts against the bottom surface wall 512 of the mounting groove 511, a farthest end of the avoidance section 820 away from the bottom surface wall 512 is higher than a position of the groove opening 514. Thus, when deflected to a maximum angle, the rotating shaft 300 is not prone to collide with the edge of the groove opening 514, reducing the risk of the rotating head 800 being jammed.

Specifically, since L3>L4, a part of the avoidance section 820 extends out of the mounting groove 511, that is, the rod portion 850 extends out of the mounting groove 511, so that a length of the entire rotating head 800 in the axial direction of the rotating shaft 300 is greater than the depth of the mounting groove 511. Specifically, in the illustrated embodiment, a stop surface 860 is formed at a side of the rod portion 850 away from the rotating head 800. The first rotor unit 410 is provided with a first shaft hole 411, a part of the rod portion 850 is sleeved in the first shaft hole 411, and the stop surface 860 abuts against a side of the first rotor unit 410 adjacent to the support member 510. Since the rod portion 850 extends out of the mounting groove 511, the rod portion 850 is located between the first rotor unit 410 and the support member 510, and a certain distance exists between the first rotor unit 410 and the groove opening 514 of the mounting groove 511, the interference and friction between the first rotor unit 410 and the support member 510 during rotation are avoided.

It should be noted that in the first embodiment, it is possible to either only provide the arc-shaped wall 513a at the groove opening 514 or only provide the avoidance section 820 at the rotating head 800, or both arrangements can be provided at the same time, which is not limited herein.

Moreover, in other embodiments, the bottom surface wall 512 can be a curved surface wall. Specifically, the bottom surface wall 512 is an outwardly convex or inwardly concave arc surface. When the bottom surface wall 512 is an outwardly convex arc surface, the rotating head 800 abuts against the outwardly convex arc surface, and the first spherical surface wall 810 is tangent to the convex arc surface. Alternatively, when the bottom surface wall 512 is an inwardly concave arc surface, the rotating head 800 abuts against the inwardly concave arc surface, and the first spherical surface wall 810 is tangent to the concave arc surface. Alternatively, when the bottom surface wall 512 is an outwardly convex arc surface, and a side of the rotating head 800 facing the bottom surface wall is a plane wall, the plane wall can also be tangent to the outwardly convex arc surface of the bottom surface wall 512.

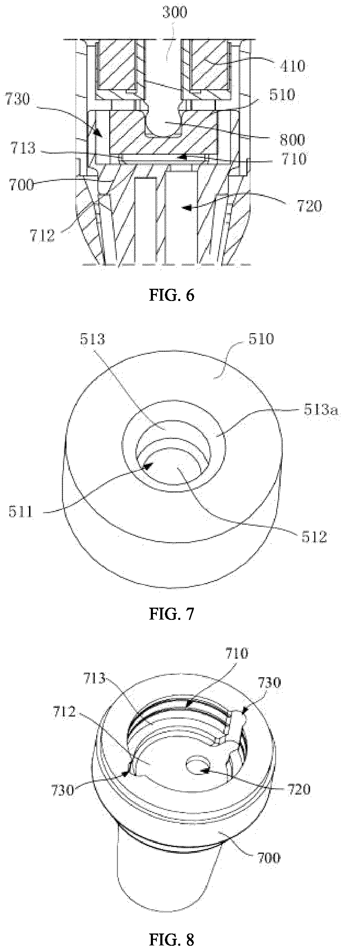

As shown in FIG. 6, FIG. 7, and FIG. 8, the driving mechanism 10 further includes a mounting base 700, and the mounting base 700 is fixedly connected to the pump case 100. The mounting base 700 includes a mounting cavity 710 and a liquid passing hole 720 communicated with the mounting cavity 710. The support member 510 is mounted in the mounting cavity 710. The mounting base 700 is provided with a flow channel. In this embodiment, the flow channel is a first flow channel 730, and the first flow channel 730 is communicated with the liquid passing hole 720, so that the flushing liquid flowing through the liquid passing hole 720 can flow into an inner cavity of the pump case 100 through the first flow channel 730. An end of the liquid passing hole 720 away from the mounting cavity 710 is configured to be communicated with the flushing line in the catheter 50, so that the flushing liquid can flow through the liquid passing hole 720 and the first flow channel 730 into the inner cavity of the pump case 100.

Specifically, one end of the first flow channel 730 is communicated with a gap between the support member 510 and a cavity bottom 712 of the mounting cavity 710, and the other end of the first flow channel 730 is communicated with the inner cavity of the pump case 100. In the illustrated embodiment, the first flow channel 730 is formed by partial recessing of a cavity wall of the mounting cavity 710. In the illustrated embodiment, two first flow channels 730 are provided, and the two first flow channels 730 are arranged opposite to each other. It can be understood that the number of the first flow channels 730 can be adjusted according to design requirements. For example, in some embodiments, the number of the first flow channel 730 can be one or more than two.

Specifically, the mounting cavity 710 has the cavity bottom 712, one opening of the liquid passing hole 720 is located at the cavity bottom 712 of the mounting cavity 710, a support step 713 is arranged in the mounting cavity 710, and the support step 713 abuts against the support member 510, so that the support member 510 is spaced apart from the cavity bottom 712 by a distance to better guarantee the flushing liquid to flow smoothly. The support member 700 is mounted in the mounting cavity 710, and the support step 713 abuts against a surface of the support member 510 facing away from the shaft sleeve 520.

Second embodiment: the second embodiment is substantially the same as the first embodiment, with the difference from the first embodiment in the structure of the rotating head 800 and the structure of the mounting groove 511.

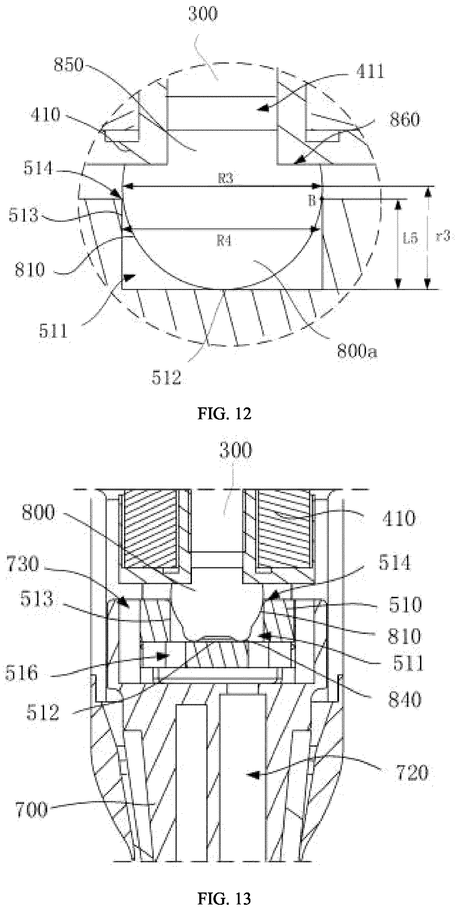

As shown in FIG. 11 and FIG. 12, the rotating head 800 includes a ball head portion 800a, the ball head portion 800a extends through the groove opening 514, and the ball head portion 800a slidably abuts against the bottom surface wall 512 and the side surface wall 513. Specifically, in the illustrated embodiment, the first spherical surface wall 810 of the ball head portion 800a is tangent to both the side surface wall 513 and the bottom surface wall 512. The side surface wall 513 of the mounting groove 511 is a cylindrical surface, the bottom surface wall 512 is a plane, and the bottom surface wall 512 is perpendicular to the side surface wall 513. The caliber of the groove opening 514 of the mounting groove 511 is smaller than or equal to the diameter of the sphere where the ball head portion 800a is located, so that a part of the ball head portion 800a is arranged outside the groove opening 514, and at least a part of the first spherical surface wall 810 is also arranged outside the groove opening 514. Therefore, the ball head portion 800a is not completely limited by the side surface wall 513 in the radial direction. With such an arrangement, when the rotating assembly 12 is deflected, the first spherical surface wall 810 can slide inside the mounting groove, and the first spherical surface wall 810 can further slide along the groove opening 514, therefore the ball head portion 800a is not limited by the groove opening 514 of the mounting groove 511 and is avoided from being stuck.

In this embodiment, the volume of the ball head portion 800a is greater than half of the sphere where the first spherical surface wall 810 is located, and the tangent point B between the first spherical surface wall 810 and the side surface wall 513 is lower than the cross section of the maximum diameter of the ball head portion 800a. That is, the tangent point B is lower than the radius of the sphere where the ball head portion 800a is located in the axial direction. The caliber of the groove opening 514 of the mounting groove 511 is smaller than the diameter of the sphere where the ball head portion 800a is located. With such an arrangement, it is possible to prevent the side surface wall 513 of the mounting groove 511 from being scratched and worn due to the contact of the side surface wall 513 of the mounting groove 511 with the included angle formed at the connection between the surface of the ball head portion 800a away from the bottom surface wall 512 of the mounting groove 511 and the first spherical surface wall 810. Such an arrangement can also cause the volume of the ball head portion 800a extending into the mounting groove 511 to be less than half of the volume of the sphere where the first spherical surface wall 810 is located. When the rotating assembly 12 is deflected, the first spherical surface wall 810 slides more smoothly in the mounting groove 511, and the risk that the first spherical surface wall is limited by the groove opening 514 to be stuck is smaller.

Specifically, the diameter of the sphere where the ball head portion 800a is located is defined as R3, the caliber of the groove opening 514 is the width of the mounting groove 511 and defined as R4, the radius of the sphere where the ball head portion 800a is located is defined as r3, and the depth of the mounting groove 511 is defined as L5. That is, R3>R4 and r3>L5. In addition, in the illustrated embodiment, the ball head portion 800a has a larger volume than that of the mounting groove 511, so that when the ball head portion is worn by the bottom surface wall 512 and the side surface wall 513, pressure points can be dispersed on the ball head portion 800a, thereby an abrasion resistance effect is good and the service life is long.

Furthermore, the caliber of the groove opening 514 of the mounting groove 511 is not smaller than half of the diameter of the sphere where the ball head portion 800a is located. That is, a minimum range of the caliber of the groove opening 514 is defined. If the caliber of the groove opening 514 is too small, the depth and width of the rotating head 800 in the mounting groove 511 are too small, and the capability of an axial or radial limitation on the rotating head 800 is too weak for high-speed rotation, and the rotating head 800 is easily separated from the mounting groove 514.

As shown in FIG. 12, the rotating head 800 includes a stop surface 860, the stop surface 860 is located on a side of the rotating head 800 away from the bottom surface wall 512 of the mounting groove 511, a distance exists between the stop surface 860 and the groove opening 514 of the mounting groove 511, and the first rotor unit 410 abuts against the stop surface 860, so that a distance exists between the first rotor unit 410 and the groove opening 514 of the mounting groove 511.

In this embodiment, the rotating head 800 includes a rod portion 850 connected to the ball head portion 800a. Specifically, the rod portion 850 is arranged separately from the rotating shaft 300, an end of the rod portion 850 away from the ball head portion 800a is fixedly connected to the first rotor unit 410, and an end of the rotating shaft 300 away from the connecting end 310 is also fixedly connected to the first rotor unit 410. In the illustrated embodiment, the first rotor unit 410 includes a first shaft hole 411, and the rod portion 850 and the rotating shaft 300 are respectively sleeved in the first shaft hole 411. In this case, the stop member 600 can be integrally formed with the rotating shaft 300, and the machining precision is good, so that the rotating shaft 300 can be assembled from a distal direction of the first shaft hole 411.

Specifically, the width of the cross section of the rod portion 850 in the direction perpendicular to the rotation axis of the rotating shaft 300 is smaller than the diameter of the sphere where the ball head portion 800a is located, the stop surface 860 is formed between the rod portion 850 and the ball head portion 800a, and the stop surface 860 abuts against a side of the first rotor unit 410 facing the support member 510, so that the stop surface has a limiting effect on the rotating head 800 during assembly. The first rotor unit 410 is spaced apart from the groove opening 514 of the mounting groove 511 by a certain distance, and the support member 510 can also be spaced apart from the first rotor unit 410, thereby effectively preventing the first rotor unit 410 from interfering and rubbing with the support member 510 during rotation.

Third embodiment: the third embodiment is substantially the same as the second embodiment, with the difference from the second embodiment in the structure of the rotating head 800 and the structure of the support member 510.

As shown in FIG. 13 to FIG. 15, the rotating head 800 further has a second spherical surface wall 840, the second spherical surface wall 840 is connected to the first spherical surface wall 810, and the rotating head 800 slidably abuts against the side surface wall 513 and the bottom surface wall 512. Specifically, the first spherical surface wall 810 is tangent to the side surface wall 513, the second spherical surface wall 840 is tangent to the bottom surface wall 512, and the second spherical surface wall 840 is arranged towards the bottom surface wall 512.

Specifically, the side of the rotating head 800 facing the bottom surface wall 512 is provided with a convex ring 830, a central axis of the convex ring 830 coincides with a rotational central axis of the rotating assembly 12, and the second spherical surface wall 840 is provided on the convex ring 830. In this embodiment, the second spherical surface wall 840 is an outwardly convex arc surface arranged around the convex ring 830. When the convex ring 830 abuts against the bottom surface wall 512, the second spherical surface wall 840 is exactly tangent to the bottom surface wall 512, so that the contact between the bottom surface wall 512 and the rotating head 800 can be regarded as a line-to-line contact, thereby reducing friction and abrasion. Since the convex ring 830 is circumferentially arranged and a contact area between the convex ring and the bottom surface wall 512 is relatively uniformly dispersed, the pressure on the portions subjected to relative friction will not be excessively concentrated, thereby further reducing abrasion and prolonging the service life of the rotating head 800.

More specifically, an inner diameter of the convex ring 830 is not less than half of the diameter of the sphere where the first spherical surface wall 810 is located. With such an arrangement, when the second spherical surface wall 840 of the convex ring 830 is tangent to the bottom surface wall 512, a perimeter of a circle defined by the convex ring 830 is large, so that the pressure applied by the convex ring 830 to the bottom surface wall 512 is dispersed and not excessively concentrated, thereby achieving a better abrasion resistance effect.

Furthermore, in other embodiments, instead of the convex ring 830, a plurality of convex balls can be arranged circumferentially and uniformly along the rotational central axis of the rotating assembly 12, and the second spherical surface wall 840 can be arranged on the convex balls, which can also achieve this effect, and the number of the convex balls can be three or four or more.

It should be noted that the structure of the convex ring 830 in the third embodiment can also be applied to the first embodiment, which is not limited herein.

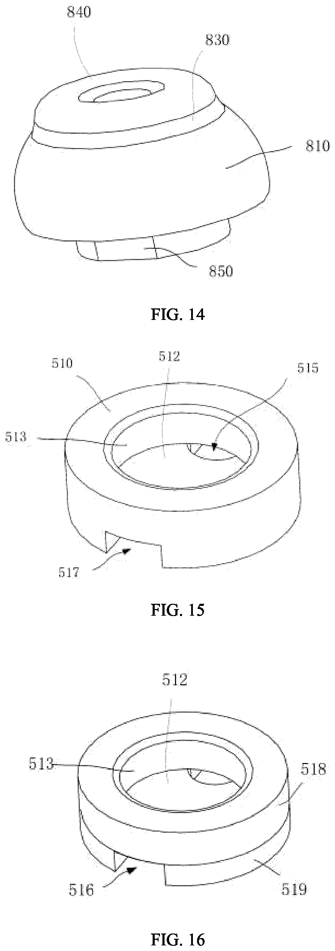

As shown in FIG. 13 and FIG. 15, in this embodiment, a flow channel is formed in the support member 510, the flow channel is a second flow channel 516, the second flow channel 516 on the support member 510 has a first opening 515 and a second opening 517, the first opening 515 is communicated with the mounting groove 511, the second opening 517 is communicated with the liquid passing hole 720, and the rotating head 800 avoids the first opening 515. Specifically, one end of the second flow channel 516 is communicated with the gap between the support member 510 and the cavity bottom 712 of the mounting cavity 710, and the other end of the second flow channel 516 is communicated with the mounting groove 511. The flushing fluid can pass through the liquid passing hole 720, the second opening 517, the second flow channel 516 and the second opening 515 and flow into the mounting groove 511. The flushing fluid enters a space between the mounting groove 511 and the rotating head 800, and can play lubricating and heat dissipation effects to reduce the friction between the rotating head 800 and the mounting groove 511 and dissipate generated heat, so as to reduce the abrasion between the rotating head 800 and the mounting groove 511. Moreover, the rotating head 800 does not cover the first opening 515 to ensure the smooth entering of the flushing liquid, and the rotating head does not contact an edge of the first opening 515 to avoid abrasion. In the illustrated embodiment, two second flow channels 516 are provided, and the two second flow channels 516 are arranged opposite to each other. It can be understood that the number of the second flow channels 516 can be adjusted according to design requirements. For example, in some embodiments, the number of the second flow channel 516 can be one or more than two.

It should be noted that the first flow channel 730 on the mounting base 700 and the second flow channel 516 on the support member 510 can be provided at the same time. Alternatively, only one of the first flow channel and the second flow channel can be provided. When both of them are arranged simultaneously, the flushing liquid enters the mounting cavity 710 via the liquid passing hole 720 and then is divided into two streams. One stream flows into the mounting groove 511 of the support member 510 through the second flow channel 516, and the other stream flows out through the first flow channel 730.

Furthermore, the support member 510 can be integrally formed or can be formed from a two-part structure. As shown in FIG. 15 and FIG. 16, when the support member 510 is formed from two parts, the support member 510 includes a collar 518 and a bottom plate 519. The bottom plate 519 is connected to an end of the collar 518. The bottom plate 519 and the collar 518 together define the mounting groove 511. At least a part of an inner wall of the collar 518 forms the side surface wall 513, and the bottom surface wall 512 is arranged on the bottom plate 519.

Specifically, the collar 518 and the bottom plate 519 can be bonded to or abut against each other. Therefore, firstly, during machining and molding, machining is easier, and the material is more freely selected. The collar and the bottom plate can be made of the same material, for example, a ceramic material. Different materials can be adopted. For example, the collar 518 is made of a ceramic material, the bottom plate 519 is made of a metal material, and a surface of the bottom plate 519 is required to be coated with a diamond coating to improve surface roughness. Secondly, during assembly, the rotating head 800 can be inserted from both axial directions of the collar 518, which is more convenient.

When the support member 510 is formed from two parts, the second flow channel 516 is arranged on the bottom plate 519, the second flow channel 516 is a notch extending from a side wall of the bottom plate 519 towards a center of the bottom plate 519, the second flow channel 516 is communicated with the liquid passing hole 720, and the rotating head 800 avoids the second flow channel 516. Alternatively, in other embodiments, the second flow channel 516 is arranged on the collar 518, the second flow channel 516 is a notch extending from a side wall of the collar 518 towards a center of the collar 518, the second flow channel 516 is communicated with the liquid passing hole 720, and the rotating head 800 avoids the second flow channel 516.

It should be noted that the structure of the support member 510 in the third embodiment can also be applied to the first embodiment and the second embodiment, which is not limited herein.

The above is only a preferred embodiment of this application and is not intended to limit this application. Any modifications, equivalents and improvements made within the spirit and principle of the present application should be included in the protection scope of the present application.

## Claims

1. A driving mechanism, comprising:
a housing assembly comprising a mounting groove and a through hole, the mounting groove having a bottom surface wall and a side surface wall; and
a rotating assembly having a far end and a near end, the far end of the rotating assembly rotatably extending through the through hole, the near end of the rotating assembly comprising a rotating head, the rotating head being rotatably arranged in the mounting groove and slidably abutting against the bottom surface wall and the side surface wall, the rotating head having a first spherical surface wall, and the first spherical surface wall being tangent to the side surface wall.

2. The driving mechanism according to claim 1, wherein a cross section of the mounting groove along a direction perpendicular to a rotation axis of the rotating assembly is in a circular shape or a rectangular shape.

3. The driving mechanism according to claim 1, wherein the side surface wall is parallel to a rotation axis of the rotating assembly.

4. The driving mechanism according to claim 1, wherein the first spherical surface wall is tangent to the bottom surface wall, and/or the bottom surface wall is perpendicular to the side surface wall.

5. The driving mechanism according to claim 1, wherein the mounting groove further comprises a groove opening and an arc-shaped wall, the rotating head extends through the groove opening, the side surface wall connects the arc-shaped wall and the bottom surface wall, and the arc-shaped wall is adjacent to the groove opening.

6. The driving mechanism according to claim 5, wherein a distance from a tangent point between the first spherical surface wall and the side surface wall to a connecting line between the side surface wall and the bottom surface wall is 60% to 75% of a distance from the groove opening to the connecting line between the side surface wall and the bottom surface wall, and a distance from the tangent point between the first spherical surface wall and the side surface wall to a connecting line between the side surface wall and the arc-shaped wall is 0.01 mm to 0.015 mm.

7. The driving mechanism according to claim 1, wherein the rotating head comprises a ball head portion and a rod portion connected to the ball head portion, the rod portion extends through a groove opening of the mounting groove, the ball head portion is rotatably received in the mounting groove, an avoidance section is formed at an end of the rod portion adjacent to the ball head portion, the avoidance section extends through the groove opening of the mounting groove, and a width of a cross section of the avoidance section in a direction perpendicular to a rotation axis of the rotating assembly is smaller than a diameter of a sphere where the ball head portion is located.

8. The driving mechanism according to claim 7, wherein the width of the cross section of the avoidance section in the direction perpendicular to the rotation axis of the rotating assembly is 75% to 85% of the diameter of the sphere where the ball head portion is located.

9. The driving mechanism according to claim 1, wherein the rotating head comprises a ball head portion, the ball head portion extends through a groove opening of the mounting groove, and a caliber of the groove opening of the mounting groove is smaller than or equal to a diameter of a sphere where the ball head portion is located and is greater than or equal to half of the diameter of the sphere where the ball head portion is located.

10. The driving mechanism according to claim 1, wherein the rotating head further has a second spherical surface wall, the second spherical surface wall is connected to the first spherical surface wall, the second spherical surface wall slidably abuts against the bottom surface wall of the mounting groove, and the second spherical surface wall is tangent to the bottom surface wall.

11. The driving mechanism according to claim 10, wherein a convex ring is further arranged on the rotating head, a central axis of the convex ring coincides with a rotation axis of the rotating assembly, and the second spherical surface wall is arranged on the convex ring.

12. The driving mechanism according to claim 11, wherein an inner diameter of the convex ring is not smaller than half of a diameter of a sphere where the first spherical surface wall is located.

13. The driving mechanism according to claim 1, wherein the housing assembly comprises a pump case and a support member mounted on the pump case, the mounting groove is formed in the support member, the driving mechanism further comprises a mounting base, the mounting base is fixedly connected to the pump case, a mounting cavity and a liquid passing hole communicated with the mounting cavity are formed in the mounting base, the support member is mounted in the mounting cavity, a flow channel is formed in at least one of the mounting base and the support member, and the flow channel is communicated with the liquid passing hole.

14. The driving mechanism according to claim 13, wherein the flow channel is formed in the support member, the flow channel in the support member has a first opening and a second opening, the first opening is communicated with the mounting groove, the second opening is communicated with the liquid passing hole, and the rotating head avoids the first opening.

15. The driving mechanism according to claim 13, wherein the support member comprises a collar and a bottom plate, the bottom plate is connected to an end of the collar, the bottom plate and the collar together define the mounting groove, at least a part of an inner wall of the collar forms the side surface wall, and the bottom surface wall is arranged on the bottom plate.

16. The driving mechanism according to claim 15, wherein the bottom plate is provided with the flow channel communicated with the mounting groove, the flow channel in the bottom plate extends from a side surface of the bottom plate towards a center of the bottom plate, and the rotating head avoids the flow channel in the bottom plate.

17. The driving mechanism according to claim 1, wherein the rotating assembly further comprises a rotating shaft and a rotor, the rotating shaft has a near end and a far end, the far end of the rotating shaft rotatably extends through the through hole, the rotor comprises a first rotor unit, the first rotor unit is fixedly connected to the rotating shaft, and at least one of the first rotor unit and the rotating shaft is connected to the rotating head.

18. The driving mechanism according to claim 17, wherein the rotating head comprises a stop surface, the stop surface is located on a side of the rotating head away from the bottom surface wall of the mounting groove, a distance exists between the stop surface and the groove opening of the mounting groove, and the first rotor unit abuts against the stop surface, so that the first rotor unit is spaced a distance from the groove opening of the mounting groove.

19. The driving mechanism according to claim 1, wherein the housing assembly comprises a pump case and a shaft sleeve mounted in the pump case, the through hole is formed in the shaft sleeve, the driving mechanism further comprises a stop member, the stop member is fixedly connected to the rotating assembly, the stop member is located between the shaft sleeve and the rotating head, and the stop member can abut against the shaft sleeve to restrict the rotating assembly from moving away from the mounting groove.

20. A blood pump, comprising an impeller and a driving mechanism, the driving mechanism comprising:
a housing assembly comprising a mounting groove and a through hole, the mounting groove having a bottom surface wall and a side surface wall; and
a rotating assembly having a far end and a near end, the far end of the rotating assembly rotatably extending through the through hole, the near end of the rotating assembly comprising a rotating head, the rotating head being rotatably arranged in the mounting groove and slidably abutting against the bottom surface wall and the side surface wall, the rotating head having a first spherical surface wall, and the first spherical surface wall being tangent to the side surface wall;
the impeller being connected to the rotating assembly, and the impeller rotating with the rotating assembly.
